# EUROPEAN PATENT APPLICATION

(11) **EP 4 691 391 A1**
(43) Date of publication of application: **11.02.2026**
(21) Application number: 24713681.5
(22) Date of filing: 20.02.2024
(51) Int. Cl.: A61B 17/42

(54) **UTERINE MANIPULATOR**

(30) Priority: 24.03.2023 ES 202330248
(71) Applicant: Universidad Jaume I De Castellón, 12071 Castellón de la Plana (Castellón) (ES); Fundación para el Fomento de la Investigación Sanitaria y Biomédica de la Comunitat Valenciana (FISABIO), 46010 Valencia (ES)
(72) Inventor: ANDRÉS DE LA ESPERANZA, Francisco Javier, 12071 Castellón de la Plana (Castellón) (ES); LLUECA ABELLA, José Antonio, 46010 Valencia (ES); FUENTES BALLESTEROS, José Feliciano, 12071 Castellón de la Plana (Castellón) (ES)
(74) Representative: Pons IP
(86) International application number: PCT/ES2024/070098
(87) International publication number: WO 2024/200889

(57) **Abstract**

The invention relates to a uterine manipulator for minimally invasive surgery that, by means of generating a vacuum area, holds the cervix along the perimeter. It comprises a tube-shaped support (1), hollow in its longitudinal axis, with a proximal end and a distal end, a vacuum tube (4), located along the hollow longitudinal axis of the support (1), which comprises, at one end corresponding to the distal end of the support (1), a vacuum chamber (5), a traction element (6), at the proximal end of the support (1) and linked to the vacuum tube (4), which pulls it with respect to the support (1), and a cup (7) located at the distal end of the support (1), with a base coupled to the vacuum chamber (5) and comprising one or more suction and drain holes (8) at the base.

## Description

### OBJECT OF THE INVENTION

The object of the present invention is a personalised uterine manipulator for minimally invasive surgery that, by means of generating a vacuum area, allows the cervix to be held along the perimeter.

### BACKGROUND OF THE INVENTION

In 1898 in Vienna, Dr. Wertheim performed the first abdominal hysterectomy to treat cervical cancer in Europe. Since then and for more than 100 years, initial cervical tumours have been treated by radical hysterectomy using an abdominal or vaginal approach.

Recently, minimally invasive techniques were described for performing said radical hysterectomies, either by laparoscopy or more recently aided by robotic assistance or laparoscopy. The incorporation of these minimally invasive techniques has been reaffirmed by multiple retrospective publications, which highlighted the advantages of minimal invasion as well as its oncological safety.

Nowadays, it is recognised that the vaginal route should be preferred to the abdominal route and that when the vaginal route is not possible, laparoscopy is better than the open route. One of the factors that has influenced greater acceptance of laparoscopic hysterectomy is the development of different surgical techniques and associated devices that improve mobilisation of the uterus during surgery and clearly identify the vaginocervical junction at the time of performing the colpotomy.

However, two recent prospective studies demonstrated a higher percentage of recurrence of these tumours, as well as deaths of patients who had undergone a laparoscopic radical hysterectomy compared to those who had undergone a classic laparotomic approach. All of this led to a modification of the most widely disseminated action guidelines for cervical cancer.

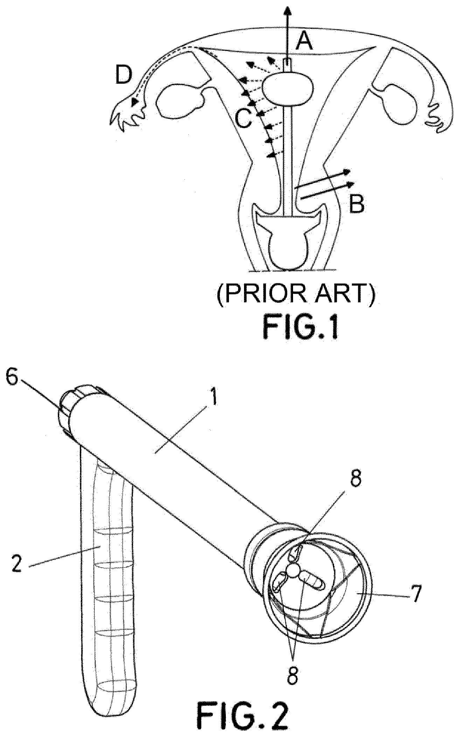

At this time, the possible causes as to why the minimally invasive approach worsens the prognosis in these patients are still not understood. Furthermore, in 2019 a retrospective analysis was carried out comparing the two approaches and found the same results but with an important particularity that gives importance to this project: it was found that patients in whom a uterine mobiliser was not used and the tumour was not manipulated had a better prognosis compared to those who used it, even if the approach was laparoscopic. Some of the hypotheses of interference between the tumour and the uterine manipulator that may explain the alteration of the myometrial barrier are shown in Figure 1.

Specifically, Figure 1 shows the macroscopic hypotheses: A, accidental uterine weakening and rupture caused by the stem of the manipulator; and B, manipulation of the tumour during insertion and colpotomy. As well as the microscopic hypotheses: an increase in pressure inside the endometrial cavity could spread malignant cells through C, lymphovascular space and D, fallopian tubes.

Most of the currently used uterine manipulator models that report less damage fall under one of the interference hypotheses cited in Figure 1, both those due to the stem (A) and those related to spreading due to the increase in pressure inside the endometrial cavity. Among them, the McCartney Tube is the only device that dispenses with the stem and maintains a simple tubular shape up to the cervix, which would supposedly reduce said damage. However, the McCartney Tube lacks any system or method of fastening between the cervix and the McCartney Tube itself while the colpotomy is performed.

Other instruments that use vacuum techniques to hold the cervix have been proposed in the patent literature, although most of them do not have performing a pericervical colpotomy as their primary objective.

Thus, document ES2755473T3 proposes a device with which the cervix is fastened using negative pressure distributed uniformly in a C- or V-shaped suction chamber. With this shape, the device allows complementary access to the external orifice of the cervix to perform gynaecological procedures such as the insertion and removal of an IUD (intrauterine contraceptive device), a uterine (endometrial) tissue swab for diagnostic purposes, cervix dilatation for uterus cavity curettage, cervix dilatation for hysteroscopy (camera in uterus), which measures uterine cavity size during surgery, hysterosalpingography (imaging procedure of the uterine cavity and fallopian tubes for fertility check-up).

However, the device is not intended for performing a hysterectomy by vaginal colpotomy, firstly, because it would be unfeasible to relocate the instrument from one region to another of the vaginal part of the cervix to continue with the colpotomy already started in a preceding region, since part of the already sectioned tissue would be detached and the anatomical shapes would not be maintained, making it impossible to fasten again by vacuum. Secondly, the device is not prepared to receive a high load of detached tissues and fluids during a colpotomy, since it has a single vacuum hole through which the suction chamber connects to a hollow rod that, if obstructed, would make the suction chamber ineffective, and it would detach from the cervix.

Document US2012283595A1 proposes a solution in which the cervix is grasped using negative pressure distributed uniformly in a hollow torus-shaped bell (cervical cup) sectioned by its coplanar plane of symmetry. The bell creates a suction chamber against an annular region of the surface of a vaginal portion of the cervix. It is primarily intended for the insertion of instruments through the centre of the torus towards the uterine canal, in other words, with a similar purpose to document ES2755473T3 analysed previously.

However, this document states as a final reflection, and not without ambiguity, that "the device may be used for any procedure which may involve manipulation of the cervix, including vaginal hysterectomy". This postulate is debatable since, constructively, the slenderness shown by the hollow rod could hardly exert bending or shear manipulation forces on the uterus, without causing deformities in the device.

These forces are inherent to the use of a manipulator, with the assisting person manipulating according to the instructions of the operating surgeon, and in favour of facilitating the colpotomy. In more specific terms related to the intended laparoscopic colpotomy, tissue detachment would easily obstruct the channel (hollow rod) that exerts the vacuum, thus losing its effect. Furthermore, the existence of a hollow torus-shaped central channel would not block the spread of cancer cells into the vagina during the procedure. In summary, it has neither been documented nor is it considered that it is used to manipulate the uterus in the vaginal hysterectomy to which its description refers.

Document WO2016018938A1 discloses a mechanism in which the distal portion includes a contact portion configured to transmit a vacuum to the surface of the cervix. As stated in the document, the cervix can be manipulated, moved and/or otherwise reoriented to facilitate the insertion of, for example, an implant or similar. It should be noted that, like the previous devices, the device disclosed in document WO2016018938A1 is also not designed to perform laparoscopic colpotomies and it does not prevent the spread of cancer cells during tissue resection. Document WO2016018938A1 clearly states that it was designed to implant devices or administer drugs.

It should be noted that none of the previous devices would be capable of performing sealing of the vagina and would not prevent the spread of tumour cells from cervical and endometrial tumours.

Document CN111374741A discloses a negative pressure cup for surrounding and fastening the cervix (without using an intrauterine stem) and isolating the diseased tissue from normal tissue, avoiding damage due to direct contact during the operation and preventing the injured cervix from coming into contact with the vagina during resection and removal, and the negative pressure suction ensures that secretions from the diseased tissue and damaged exudate do not escape.

Documents US3926192A, CN209253065U and US2011130769A1 disclose uterine manipulators, all of which have a manipulator stem that can spread malignant cells to both the abdomen and the vagina.

In the first document (US3926192A), although the device is disclosed for a particular type of surgical procedure (laparoscopic tubal fulguration), it is indicated that the instrument can be used in other situations where deliberate direction and movement of the uterus is required. It also asserts that the cervical cup can be vacuum-fastened to the uterine wall surrounding the cervical orifice, although constructively only a thin conduit connects the free space of the cup to the vacuum tube. This can lead to the aforementioned problems, associated with the obstruction of said conduit.

The cup of the device disclosed in US3926192A is inserted vaginally and is not able to fold, thus foreseeing certain difficulties and damage to the vaginal canal. Furthermore, the threaded portion on which the stem is screwed is at the level of the external edge of the cup, which makes it difficult for the entire edge of the cup to be in simultaneous contact with the vaginal wall of the cervix and therefore makes it difficult to achieve sealing that ensures the vacuum.

The second document (CN209253065U) also offers the option to remove the stem. In any case, the support of said stem crosses and occupies the entire longitudinal axis of the instrument, not offering the option to use vacuum techniques. In this device, the cup is obtained through a 3D printing preparation preferably using a powder nylon material as a printing consumable. It is also suggested that the mouth of the cup be thicker than its bottom. Thus, given the shape of the cup section and the material used (nylon), it is expected that its rigidity and dimensions will cause difficulty and damage when inserting it through the vaginal canal.

Finally, the third document (US2011130769A1) is intended for laparoscopic-assisted vaginal hysterectomy. The proposed device has a distally open bell that receives the cervix, and a seal (on the proximal side of said bell) that, like an inflatable, performs sealing of the vagina and thus maintains pneumoperitoneum, preventing the collapse of the abdomen during laparoscopy. The suction bell and the sealing bell are designed as a unit or at least part of a unit securely fastened on the stem. The suction bell is designed in a stepped manner on its inner side, to ensure that the vaginal portion of the cervix (portio) is inserted in a tight and firm manner against different anatomical sizes of the portio, along the entire periphery. This unit is proposed as a (disposable) consumable provided in sterile packaging, whereby it is only connected to the instrument directly before use and it is discarded after the operation, after removal.

The device consists of a hollow intrauterine stem that extends through the instrument and forms a distal section that is used to manipulate the uterus, and to aspirate intrauterine cells. Additionally, at least one suction channel opens into the bell so that said bell is subjected to a vacuum with a dual effect: to aspirate any cancer cells externally in the portio region, and to be used to fasten the uterus in the manipulator. Thus, with this uterine manipulator, traction forces can be applied from the outside without there being the danger of the stem sliding out of the cervical canal (and a mechanical anchorage inside the uterus, commonly an inflatable intrauterine balloon, is dispensed with).

The body of the device, in its proximal portion, is thickened to perform the functions of a handle, and there is a spring-loaded locking body inside the handle, so that this locking body fits into one of the notches and thus, depending on the selection of the notch, fastens the axial extension from the handle to the distal end of the stem.

One of the advantages that it asserts is that the suction bell is made of a rigid material (e.g., preferably transparent injection moulded plastic), while the vaginal seal is softelastic (e.g., a silicone part), at least in the peripheral region.

This third document (US2011130769A1) has the following disadvantages: (i) as in the previous cases, it uses an intrauterine stem during the operation, (ii) it uses a rigid cup that, as mentioned above, makes its insertion difficult, and (iii) it is operated with a handle arranged longitudinally on the body, with a locking mechanism for the intrauterine stem, which furthermore complicates assembly in the operating room and sterilisation of these parts. Moreover, it does not clarify any speculation regarding the possibility of obstructing the vacuum channel, which is the only channel and has little space as it is shared by the stem.

### DESCRIPTION OF THE INVENTION

The object of the present invention is a personalised uterine manipulator for minimally invasive surgery. The manipulator promotes an operating technique that differs from the technique used by the manipulators of the state of the art by opting for a vacuum as the only means of holding the cervix during colpotomy, in other words, completely dispensing with the intrauterine stem that the devices of the state of the art have for such an operation.

Specifically, the uterine manipulator firstly comprises a hollow cylindrical support, provided with a proximal end and a distal end intended to be inserted into the vaginal canal. The diameter of the support is sized so as not to be critical in the vaginal canal. There is preferably a handle at the proximal end to facilitate the use of the manipulator.

In one aspect of the invention, the manipulator may comprise an inflatable ring arranged around the support, which safeguards the pneumoperitoneum (of the colpo-pneumo-occluder type (Cooper-SurgicalTM), or similar) when the support is inserted into the vaginal canal.

Secondly, the manipulator comprises a vacuum tube, which is located inside the support, and there is a vacuum chamber at a distal end of the vacuum tube.

Thirdly, the manipulator comprises a traction element, arranged at the proximal end of the support, which allows the proximal end of the vacuum tube to be pulled with respect to the support. The traction element may be a nut, preferably with a square or trapezoidal thread, which is linked to a threaded region located at a proximal end of the vacuum tube, in correspondence with the proximal end of the support.

Fourthly, the uterine manipulator comprises a cup, located at the distal end of the support. The cup comprises an open region, intended to hold the cervix along the perimeter, and a base on which the vacuum chamber is coupled. The vacuum chamber has suction and drain holes, preferably three. In this way, the cup remains as a diaphanous space inside of which the vacuum that holds the portio of the cervix along the perimeter is generated.

The suction and drain holes enable this vacuum to be generated while facilitating the drainage from the cup of any liquid that may be generated, which circulates through the vacuum tube and is removed to the outside without ever coming into contact with the vaginal canal. Furthermore, the suction and drain holes enable the functionality to be maintained while the colpotomy is performed, since they prevent the loss of the vacuum inside the cup.

Furthermore, as indicated, there will be preferably three suction and drain holes, since this way the chamber distributes the suction effect between them, while ensuring that, in the event that one or more of them become obstructed, the negative pressure inside the cup is not lost.

### DESCRIPTION OF THE DRAWINGS

As a complement to the description provided and for the purpose of helping to make the features of the invention more readily understandable, in accordance with a practical preferred exemplary embodiment thereof, said description is accompanied by a set of drawings which, by way of illustration and not limitation, represent the following:
Figure 1 shows a representation of possible macroscopic and microscopic hypotheses.
Figure 2 shows a general view of the uterine manipulator of the invention.
Figure 3 shows a section of the assembled uterine manipulator.
Figure 4 shows a section of the disassembled uterine manipulator.
Figure 5 shows a sectional view of the manipulator during a colpotomy.
Figure 6 shows a perspective view of the cup of the uterine manipulator.

### PREFERRED EMBODIMENT OF THE INVENTION

A preferred embodiment of the uterine manipulator, object of the present invention, is described below with the aid of figures 1 to 6.

As shown in figure 2, the uterine manipulator firstly comprises a hollow cylindrical support (1) provided with a distal end that ends in a thickened area and a proximal end in which a handle (2) is located, which will be used to help the specialist use the manipulator easily. The support (1) is intended to be inserted into the vaginal canal through the distal end.

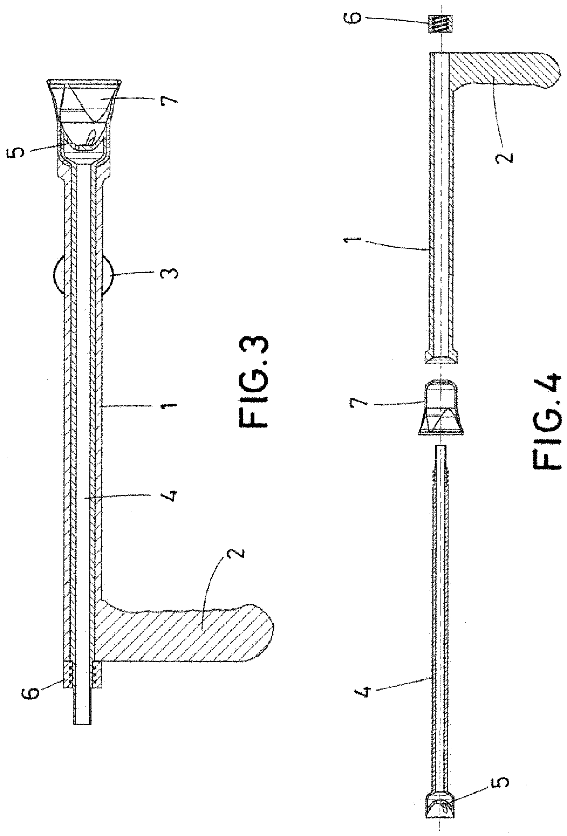

As shown in figure 3, the manipulator may comprise an inflatable ring (3) made of medical grade silicone and preferably single-use, arranged around the support (1), which safeguards the pneumoperitoneum (of the colpo-pneumo-occluder type (Cooper-SurgicalTM), or similar) when the support (1) is inserted into the vaginal canal.

Secondly, as appears in detail in figure 3, the manipulator comprises a vacuum tube (4), located inside the hollow cylindrical support (1), which ends in a vacuum chamber (5) at its distal end.

Thirdly, at the proximal end of the vacuum tube (4), in correspondence with the proximal end of the support (1), the manipulator comprises a traction element (6), which is reflected in figure 4. The traction element (6) may be a nut, with a square or trapezoidal thread, which is linked to a threaded region of the vacuum tube (4), located in correspondence with the proximal end of the support (1). The traction element (6) enables the vacuum tube (4) to be pulled with respect to the support (1) when the traction element (6) is tightened against the support (1).

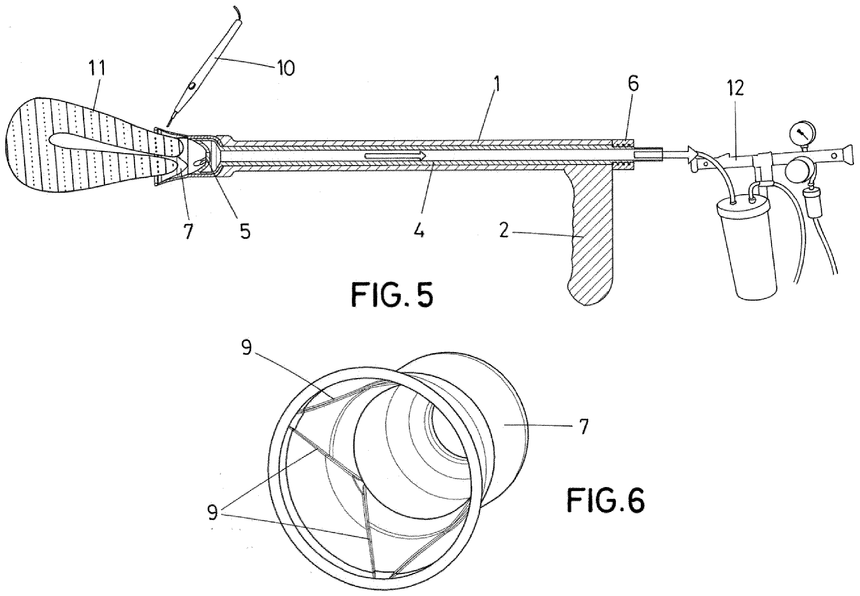

Fourthly, the uterine manipulator comprises a cup (7), shown in detail in figures 2 and 6, which is provided with a base and an open end intended to be coupled to a cervix (11), in which the base is located on the thickened area of the distal end of the support (1), it is crossed by the vacuum tube and it is in turn held by the vacuum chamber (5) of the vacuum tube (4),

The base of the vacuum chamber (5) has suction and drain holes (8), preferably three. The sealing of the assembly of the cup (7) with the vacuum chamber (5) is guaranteed by the tightening of the vacuum chamber (5) against the distal end of the support (1) with the cup (7) therebetween, when the vacuum tube (4) is pulled by the action of the traction element (6).

As reflected in figure 5, the proximal end of the vacuum tube (4) is connected to a vacuum and fluid purge module (12), which holds the cervix (11) with the cup, at the same time that it removes any fluid that may be generated, preventing it from coming into contact with the vaginal canal. Thus, while the holding of the cervix (11) is ensured, the specialist can perform the necessary operations with an electroscalpel (10) as shown in said figure 5.

The support (1), the handle (2), the traction element (6) and the vacuum tube (4) are made of a lightweight, rigid and sterilisable material, preferably nylon plastic or ABS, so that they can be reusable. As in other manipulators, the total length of the cylindrical support (1) and the vacuum tube (4) far exceed the length of a vagina, so the nominal measurements can be a standard.

For its part, the cup (7) is made of a flexible material, and can be printed in 3D, in TPU or similar, in a size system that enables choosing the most appropriate size for the patient or doing so in a personalised way, in other words, in such a way that the shape and dimensions of the cup (7) can be personalised based on the measurements and shape that are recognised and segmented from the patient's medical image (MRI, CT).

Furthermore, the cup (7) is made of a flexible material to facilitate its folding and insertion into the vaginal canal. However, the geometry and thickness given to its wall enables it to withstand sufficient negative pressure to adhere to and hold the portio of the cervix (11) during colpotomy, as shown in figure 5.

In one aspect of the invention, shown in detail in figure 6, the cup (7) comprises, on its inner face, radial patterns of triangular regions (9) having different thicknesses that extend from the open end of the cup towards the base, guaranteeing structural rigidity against the effect of internal negative pressure (through the thickest regions), but facilitating the manual folding of the cup (7) for its insertion through the vaginal canal (through the thinnest regions).

Lastly, the cup (7) may comprise a surface coating layer of virucidal agents, with a photosensitising nature, capable of generating reactive oxygen species, which can be activated during the surgical procedure using UV.

## Claims

1. A uterine manipulator, **characterised in that** it comprises:
- a hollow cylindrical support (1) provided with a proximal end and a distal end that ends in a thickened area,
- a vacuum tube (4), located inside the support (1) provided with a proximal end and a distal end,
- a vacuum and fluid purge module (12) connected to the proximal end of the vacuum tube (4),
- a vacuum chamber (5), defined at the distal end of the vacuum tube (4), comprising one or more suction and drain holes (8)
- a traction element (6), located at the proximal end of the support (1) and linked to a threaded region defined in the proximal end of the vacuum tube (4), which it pulls with respect to the support (1), and
- a cup (7), with a base and an open end intended to be coupled to a cervix (11), in which the base is located on the thickened area of the distal end of the support (1), it is crossed by the tube vacuum and it is in turn held by the vacuum chamber (5) of the vacuum tube (4).

2. The uterine manipulator of claim 1, wherein the traction element (6) is a square or trapezoidal nut.

3. The uterine manipulator of claim 1, additionally comprising a handle (2) at the proximal end of the support (1).

4. The uterine manipulator of claim 1, wherein there are three suction and drain holes (8).

5. The uterine manipulator of claim 1, additionally comprising an inflatable ring (3) arranged around the support (1).

6. The uterine manipulator of claim 5, wherein the inflatable ring (3) is made of medical grade silicone.

7. The uterine manipulator of claim 1, wherein the support (1), the traction element (6) and the vacuum tube (4) are made of a material selected from nylon plastic and ABS (acrylonitrile butadiene styrene).

8. The uterine manipulator of claim 3, wherein the handle (2) is made of a material selected from nylon plastic and ABS.

9. The uterine manipulator of claim 1, wherein the cup (7) is made of a flexible material.

10. The uterine manipulator of claim 1, wherein the cup (7) comprises, on its inner face, radial patterns of triangular regions (9) having different thicknesses that extend from the open end of the cup towards the base.

11. The uterine manipulator of claim 1, wherein the cup (7) comprises a surface coating layer of virucidal agents, with a photosensitising nature.
